(19) Europäisches Patentamt
European Patent Office
Office européen des brevets

(11) Publication number: **0 350 320 A2**

# (12) EUROPEAN PATENT APPLICATION

(21) Application number: 89306907.0

(22) Date of filing: 07.07.89

(51) Int. Cl.[5]: C 07 B 35/06
C 07 C 49/203,
C 07 C 49/205,
C 07 C 49/175,
C 07 C 49/255,
C 07 C 59/215, C 07 C 69/67,
C 07 C 69/73, C 07 C 255/09,
C 07 C 255/12, C 07 C 255/17

(30) Priority: 07.07.88 GB 8816239

(43) Date of publication of application:
10.01.90 Bulletin 90/02

(84) Designated Contracting States:
AT BE CH DE ES FR GB GR IT LI LU NL SE

(71) Applicant: PHARMAGLOBE LABORATORIES LTD
100 Westmore Drive Unit 8
Rexdale Ontario M9V 5C3 (CA)

(72) Inventor: Gyuran, John Janos
35 Selan Crescent
Rexdale Ontario M9V 1N8 (CA)

(74) Representative: McCall, John Douglas et al
W.P. THOMPSON & CO. Coopers Building Church Street
Liverpool L1 3AB (GB)

## (54) Production of unsaturated compounds.

(57) Unsaturated compounds, in particular alkenyl ketones and cyano analogues, useful for polymerization with a variety of monomers to form photodegradable polymers, are formed by dehydrating a preformed liquid alkanol of the formula:

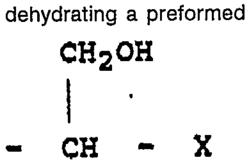

(I)

where X is an activating group for this dehydration reaction, preferably -CO- or -CN, in the presence of a solid dehydration catalyst, which may be acid resin, alumina or mixtures thereof. Apparatus for carrying out the method and novel products also are described.

EP 0 350 320 A2

## Description

# PRODUCTION OF UNSATURATED COMPOUNDS

## FIELD OF INVENTION

The present invention relates to the production of unsaturated compounds, particularly to the production of alkenyl ketones and cyano analogues useful for polymerization with a variety of monomers to form photodegradable materials and to certain novel alkenyl ketones, alkenyl cyano compounds, alkenyl esters and alkanol analogs.

## BACKGROUND TO THE INVENTION

Unsaturated ketones, such as methyl isopropenyl ketones (mipk), are useful in a number of commercial applications, including as monomers for copolymerization with other free radically polymerizable ethylenically-unsaturated monomers, such as ethylene, styrene, butadiene, isoprene and methyl methacrylate, to form specialty rubbers and plastics. The presence of ketone carbonyl groups in the polymer chain provides photodegradability to the polymers upon exposure to ultraviolet light, such as is contained in direct sunlight. Such polymers and condensation polymers incorporating such ketone carbonyl groups have been the subject of a number of patents, including Canadian Patents Nos. 975,491, 975,492, 983,200, 987,972, 994,950, 1,000,000 and 1,180,793.

Mipk and analogues, such as methyl vinyl ketone, are difficult and somewhat dangerous to handle. They pose health problems as lacrimatory substances and need to be prepared and handled with extreme care. In addition, transportation in bulk over large distances is hazardous, for fear of accidental spillage as well as the inherent danger in their transfer between vessels, containers and storage equipment.

Procedures used to form mipk and its analogues generally involve reaction of formaldehyde with methyl ethyl ketone in the presence of a basic catalyst, such as potassium hydroxide, to form 2-methyl-3-ketobutan-1-ol, followed by dehydration thereof using phosphoric acid, hydroquinone or copper powder.

The dehydration is carried in a series of batch processes. The yield is about 45 to 50% as a result of polymerization of mipk in the long process (typically 4 to 5 hours). The process also generates acidic water, which exceeds the amount of mipk generated, resulting in environmental problems.

Another prior art procedure is a continuous one in which an aqueous solution of methyl ethyl ketone in water is pumped through a packed catalyst bed, but the yield in this case is only about 5 to 10% mipk.

The product mixtures from these procedures have to be separated by fractional distillation to isolate the mipk. The prior art processes expend a considerable amount of energy, not consumed in the process of the invention and also generate a considerable amount of waste by-products, above five times the amount generated by the process of the invention.

## SUMMARY OF THE INVENTION

In accordance with the present invention, there is provided a novel process for the formation of alkenyl ketones and cyano analogues, which is capable of formation of desired product in high yield with little residue, which is susceptible of continuous operation and which is capable of producing a wide range of products, as well as certain novel compounds.

In accordance with one aspect of the present invention, a preformed liquid alkanol of the general formula (I):

$$\begin{array}{l} CH_2OH \\ \ | \\ -CH-X \end{array} \qquad (I)$$

where X is an activating group for the methylolation and dehydration reactions, is dehydrated by heating the same in the presence of a solid dehydration catalyst which is an acid resin, a mixture of an acid resin and alumina, alumina, a mixture of alumina and a heavy metal oxide, or a mixture of acid resin, alumina and heavy metal oxide.

The product mixture, which contains a high yield of the desired unsaturated compound, water and unreacted alkanol, may be subjected to evaporative separation to separate vaporized product unsaturated compound and water from unreacted alkanol, which may be recovered and recycled, followed by condensation of the vaporized product and water and separation of liquid desired product from condensed water.

The product mixture generally contains about 70 to about 95% of the desired unsaturated compound,

about 1 to about 15% of water and about 30 to about 1% unreacted alkanol.

In accordance with a second aspect of the present invention, there are provided certain novel alkanols and certain novel unsaturated compounds. The novel alkanols provided in this aspect of the invention have the formula:

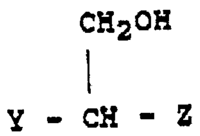

wherein Y represents $R_1CO$-, in which $R_1$ represents a lower alkyl group or a halogen atom, or CN- and, (a) when Y is $R_1CO$-, Z represents -CO-$R_2$ in which $R_2$ represents a hydrogen atom, a halogen atom, or an -$OR_3$ group in which $R_3$ represents a lower alkyl group, (b) when Y is -CN, Z represents a cyano group or a $R_4CO$-group in which $R_4$ represents a lower alkyl group or an-$OR_3$ group in which $R_3$ represents a lower alkyl group.

## BRIEF DESCRIPTION OF DRAWING

Figure 1 is a schematic representation of one embodiment of an apparatus for carrying out the preparative method of the invention.

## GENERAL DESCRIPTION OF INVENTION

The process of the present invention is applicable to a wide range of liquid alkanols of the general formula (I):

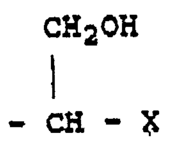

(I)

where X is an activating group for the dehydration reaction, which forms a corresponding wide range of unsaturated reaction products of the general formula (II):

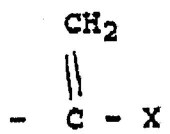

(II)

In one group of compounds of this formula (I), the X group is a carbonyl group. Representative compounds of such group are those of the formula:

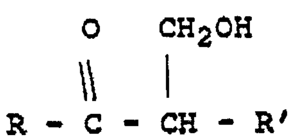

where R represents a lower alkyl group, preferably a primary, secondary or tertiary alkyl group containing from 1 to 6 carbon atoms, which may be substituted by a halogen atom, preferably chlorine; a halogen atom; preferably chlorine; an amino group; or an -$OR_3$ group, where $R_3$ represents a lower alkyl group, preferably a primary, secondary or tertiary alkyl group containing from 1 to 6 carbon atoms, which may be substituted by a halogen atom, preferably chlorine, and R′ represents hydrogen, a lower alkyl group, preferably a primary, secondary oer tertiary alkyl group containing from 1 to 6 carbon atoms, which may be substituted by a halogen atom, preferably chlorine, or an -$OR_3$ group, which produce unsaturated ketones of the formula:

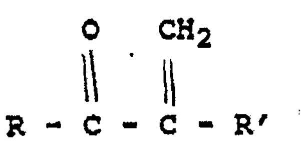

of which methyl isopropenyl ketone and methyl vinyl ketone are representative examples.

Other representative compounds of such group are those of the formula:

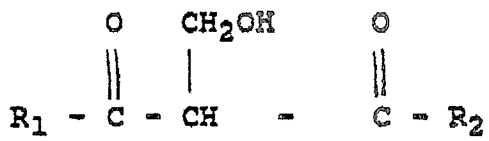

where $R_1$ represents a lower alkyl group, preferably a primary, secondary or tertiary alkyl group containing from 1 to 6 carbon atoms, which may be substituted by a halogen atom, preferably chlorine; or a halogen atom, preferably chlorine; an amino group; an $-OR_3$ group, where $R_3$ represents a lower alkyl group, preferably a primary, secondary or tertiary alkyl group containing from 1 to 6 carbon atoms which may be substituted by a halogen atom, preferably chlorine; and $R_2$ represents a hydrogen atom; a halogen atom, preferably chlorine; an amino group; a lower alkyl group, preferably a primary, secondary or tertiary alkyl group containing from 1 to 6 carbon atoms, which may be substituted by a halogen atom, preferably chlorine; an $-OR_3$ group, which produce the corresponding unsaturated compounds:

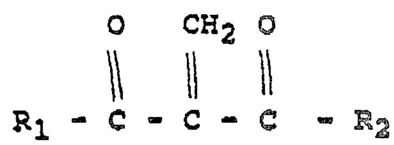

Of the latter compounds, those in which $R_1$ is a lower alkyl group or a halogen atom, and $R_2$ is a hydrogen atom or halogen atom; and those in which $R_1$ is a lower alkyl group, and $R_2$ is an $-OR_3$ group, are novel compounds, as well as the corresponding alkanols.

In another group of compounds of the formula (I), the X group is a cyano group. Representative compounds of such group are those of the formula:

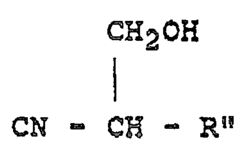

wherein R″ represents a cyano group, or a $R_4CO-$ group where $R_4$ represents a lower alkyl group, preferably a primary, secondary or tertiary alkyl group containing from 1 to 6 carbon atoms, which may be substituted by a halogen atom, preferably chlorine, or an $-OR_3$ group where $R_3$ is a lower alkyl group, preferably a primary, secondary or tertiary alkyl group containing from 1 to 6 carbon atoms, which may be substituted by a halogen atom, preferably chlorine, which produce the corresponding unsaturated compounds:

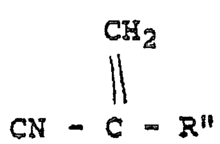

These are novel compounds as are their precursor alkanols.

Accordingly, the present invention enables the production of a wide class of unsaturated compounds to be achieved, including unsaturated ketones, unsaturated diketones, unsaturated ketoesters, unsaturated ketonitriles, unsaturated ketonitrile esters, unsaturated diesters, unsaturated ketoaldehydes and unsaturated dinitriles.

The solid catalyst employed in the dehydration reaction may comprise an acid ion exchange resin, which may be provided in admixture with alumina. Such acidic ion exchange resins may be any of the known ones of such resins, including those sold under the trademark "AMBERLITE" and "DIAION". Preferably, the catalyst comprises a mixture of acid resin and alumina in the weight proportions of about 90:10 to about 50:50 resin to alumina.

Alumina alone also may be used as the catalyst. The alumina, when used alone or in combination with the resin, may also contain a minor amount of at least one heavy metal oxide. A catalyst comprising a major portion of alumina and a minor portion of heavy metal oxide, generally containing about 97.5 to about 99.75 wt.% $Al_2O_3$, may be used in the dehydration process.

Heavy metal oxides which may be used include iron oxide, nickel oxide, cobalt oxide, titanium dioxide, vanadium oxides, manganese oxides, copper oxides, zinc oxide, zirconium oxides, molybdenum oxide, palladium oxide, platinum oxide and tungsten oxide. The preferred heavy metal oxide is iron oxide.

The mixed metal oxide catalyst conveniently can be prepared by a coprecipitation process from a mixture of the corresponding metal salts. For example, to prepare the preferred mixed catalyst, aluminum trichloride and ferrous chloride may be mixed in the appropriate proportions, their hydroxides formed and precipitated by the addition of an aqueous solution of ammonium hydroxide or sodium hydroxide. The precipitate is filtered off,

washed, sieved to obtain catalyst particles of desired size range and then dried and activated by heating in oxygen or air at temperatures of up to about 200°C, to form the desired dehydrated metal oxide catalyst.

The mixture of acidic ion exchange resin and alumina may be formed by mixing the resin with the alumina oxide or aluminum oxide and heavy metal oxide mixture, having substantially the same particle size as the resin, made by the method described above.

The starting material alkanol preferably is provided with a high degree of purity, particularly with respect to water content, prior to contact with the dehydration catalyst, so as to minimize the water and starting material content of the unsaturated reaction product mixture formed. In this regard, it is preferred that the starting alkanol have a purity of at least about 90% and a water content of less than about 2%. The alkanol preferably is prepared by reacting the corresponding reactant in substantially anhydrous form with substantially anhydrous formaldehyde or paraformaldehyde, in the presence of a basic catalyst, under substantially anhydrous conditions.

The dehydration reaction is effected by bringing the alkanol into contact with the catalyst in a suitable reaction chamber at an elevated temperature, generally the range of about 80° to about 200°C, preferably about 130° to about 150°C. The reaction may be effected by flowing the alkanol in liquid form over a fixed bed of the catalyst in an elongate reaction chamber, so that dehydration occurs as the alkanol flows through the reaction chamber.

The reaction products from the dehydration reaction are the corresponding unsaturated compound and water. This mixture is subjected to evaporative separation promptly after formation, at a temperature generally in the range of about 80° to about 120°, preferably about 90° to about 100°, under appropriate pressure conditions, to effect evaporation of the water and unsaturated compound from the product mixture while leaving unreacted alkanol in the liquid phase. The residual unreacted alkanol may be recycled to the dehydration step.

The evaporated unsaturated product and water are condensed and form two liquid phases, which are readily separated one from the other, such as by decantation.

The overall process may be effected in continuous manner to produce the unsaturated product in high yield and in high purity. The handling requirements are minimal. The process of the invention may be operated in tandem with a polymerization unit wherein the unsaturated compounds produced by the method of the invention may be homo- or co-polymerized, thereby eliminating the hazardous handling and possible spillage during transportation.

One advantage that the present invention possesses is that a stabilizer need not be added to the product, in contrast to the prior art products. In the prior art, such stabilizer is required and must be removed before polymerization can occur.

## DESCRIPTION OF PREFERRED EMBODIMENT

Referring to the drawing, there is shown therein one embodiment of a dehydrating and recovery apparatus 10 for dehydrating alkanols to form the corresponding unsaturated compounds. The construction and operation of the dehydrating and recovery apparatus will be described with respect to the formation of methyl isopropenyl ketone. It will be understood, however, that the procedure described apply equally to the production of the wide range of unsaturated compounds described above.

Pre-prepared 2-methyl-3-ketobutan-1-ol, substantially water-free, is stored in bulk in vessel 12, under agitation, and at a temperature of about 50° to about 90°C, preferably about 50° to about 70°C, maintained by an appropriate heating coil 14 therein. The ketobutanol is pumped via pump 16 to a heating unit 18 which contains circulating heat transfer fluid therein passing through. The ketobutanol passes through a coil 20 within heating unit 18, in which its temperature is raised to about 110° to about 150°C, typically about 130°C.

The heated ketobutanol passes via line 22 to a jacketed packed bed reactor 24 where it encounters a bed of solid catalyst, for example, alumina. Heat transfer fluid is circulating through the jacket 26 of the reactor 24, to maintain the catalyst therein at about 110° to about 160°C, typically about 150°C, for the dehydration reaction. The reaction is maintained under pressure, generally about 5 to about 10 bar, preferably about 6 bar.

The reaction products and unchanged ketobutanol exit the reactor 24 via line 28 and pressure control valve 30 through which the pressure is let down to approximately atmospheric, into a liquid-vapor flash separator 32, at a temperature of between about 90°C and about 110°C. At this temperature, the unreacted ketobutanol as a liquid condenses at 34, to be temperature controlled therein at a temperature of about typically about 105°C to maintain it in liquid phase, by coil 36. The bottom of separator 32 communicates via flow valve 38 with a recycle storage vessel 40, from which the ketobutanol can return via pump 42 and line 44 to vessel 12, or alternatively via pump 42 and line 46, to waste.

The mixture of mipk and water vaporizes in flash separator 32 and exists via top port 48 into water cooled condenser 50, wherein the mixture is condensed to liquid phase, and collected in a continuous decanter 52. In the decanter 52, the liquid mixture phase separates into an upper mipk layer 54, which can be continuously decanted by overflow line 56 to storage vessel 58, and a lower water layer 60. A water storage vessel 62 receives water drained from continuous decanter 52. The various liquid holding tanks 12, 40, 58 and 62, as well as the continuous decanter 52, are vapor vented to a vent gas condenser 64 equipped with a refrigerant cooling coil 66, to condense any volatile vapors. The upper gas outlet 68 from the condenser 64 is connected

to a catalytic vented gas scrubber 70, to clean up any vent gasses before they are discharged to atmosphere. The vent may be attached to a vacuum pump to operate the region of the apparatus following the check valve 30 under subatmospheric pressure, if desired.

By means of the procedure described with respect to apparatus 10, mipk is produced and stored in vessel 58 in a sufficiently high degree of purity for direct feed to a polymerization unit, without need for further purification. The product is at least about 95% pure, and is produced simply and economically in the reactor unit 10. The unit 10 may be constructed to be mobile and portable, for temporary or permanent integration into an alkenyl ketone polymerization or copolymerization unit.

## EXAMPLES

The invention is illustrated further by the following Examples, in which Examples A, B and C contain preparative procedures for forming starting material methylols and Examples 1 to 10 are illustrative of the present invention.

EXAMPLE A

This Example describes the preparation of 4-hydroxy-3-methyl-2-butanone (Methylol).

In a 2 L round bottom flask, fitted with magnetic stirrer and in an oil bath, 1.35 L of methyl ethyl ketone (MEK) were placed. While stirring, 90 g of paraformaldehyde were slowly added, followed by 7.5 mL of a 0.5 N ethanolic solution of KOH.

A condenser was placed on top of the flask and the mixture was warmed to 40°C, at which point the heat was turned off. Following initiation of the reaction in this way, reaction continued until an isotherm is reached, thus indication completion of reaction. The reaction mixture warms to about 50°C.

The completion of the reaction was monitored with Tollens reagent (for aldehyde detection) and when the test was negative, the reaction was finished, in about one hour.

3.0 L of 2 N acetic acid in MEK was added and the mixture was stirred for 3 minutes and then set up for reduced pressure distillation in the same flask. The reduced pressure distillation was considered finished when MEK content was less than 1%, which can be monitored by GC analysis. Oil temperature was maximum 110°C (methylol boils at 90° to 95°C (15 mm Hg).

The residue in the flask was the methylol and its appearance was that of a yellowish viscous liquid. The yield was about 260 g, which represents about 90% of the theoretical, based on paraformaldehyde.

EXAMPLE B

This Example describes the preparation of monomethylolmalonicacidethylester.

At room temperature, 114 mL (0.75 moles) of diethyl malonate was dissolved in 600 mL of ethanol, and the pH of the mixture was adjusted to 8, using 4.5 N KOH in methanol (about 3.5 mL). 22.5 g (0.75 moles) of paraformaldehyde then was added to the mixture which was then maintained at room temperature (20° to 25°C) for 8 hours. When all of the paraformaldehyde had reacted, as determined by testing for the aldehyde content of the reaction mixture with Tollens reagent, the pH was adjusted back to 6, by adding 2 N acetic acid in ethanol, and excess ethanol was evaporated off under reduced pressure. The residual monomethylolmalonicacidethyl ester, a syrup, weighed about 133 to 136 g, a crude yield of about 93 to 95%.

EXAMPLE C

This Examples describes the preparation of 3-methylol-2,4-pentanedione, a new compound.

In a 500 mL 3-neck round bottom flask, in an oil bath, and fitted with thermometer and stirring bar, 200 mL methanol and 26 mL of 2,4-pentanedione were placed and 0.25 mL of 4.5 N KOH in methanol was added dropwise to a pH of 8 to 8.2. When the desired pH was reached, 7.5 g of paraformaldehyde was added and the reaction mixture was stirred at room temperature for about 10 hours. The paraformaldehyde was completely dissolved and presence of aldehyde was checked using Tollens test. When the test was negative, the mixture was cooled and acidified by adding, dropwise, about 2.0 mL of 2 N acetic acid, to the point where pH is 6 to 6.5. Methanol was then evaporated and resulting syrup 3-methylol-2,4-pentadione, usually weighs about 30 g (92% yield).

An analytical sample of the crude product was prepared by distilling the crude material in high vacuum (0.1mm Hg) at 80°C, repeatedly three times, and was analyzed

| | | | |
|---|---|---|---|
| Theoretical: $C_6H_{10}O_3$ | C-55.7% | Found | C-55.49% |
| | H-7.75% | | H-7.50% |
| | O-36.88% | | O-37.01% |

The good correlation of determined carbon, hydrogen and oxygen contents of the product demonstrated that the product was the expected one.

**EXAMPLES**

Similar preparative procedures to those described in Examples A to C are employed to form other methylol

compounds suitable for dehydration to the corresponding alkenyl compounds following the procedures outlined in Examples 1 to 10.

The following Table I lists the commercially available starting material, the methylol compound formed by reaction with paraformaldehyde use the corresponding alkenyl compound produced by dehydration of the methylol compound. Where the compound is considered new, it is so indicated:

TABLE I

| Starting Compound | Methylol Compound | Alkenyl Compound |
|---|---|---|
| $CH_3$-CH-CN<br>Acetyl Acetonitrile | 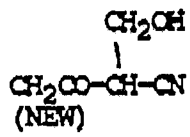<br>(NEW) | 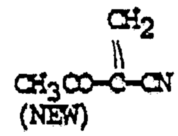<br>(NEW) |
| 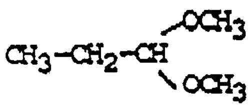<br>3-keto-butyraldehyde-dimethyl acetate | 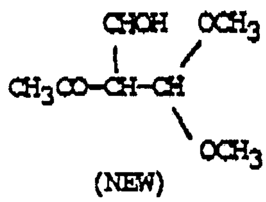<br>(NEW) | 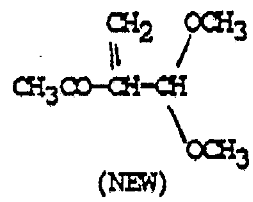<br>(NEW) |
| $CH_3$-$CH_2$-COCl<br>Aceto acetylchloride | 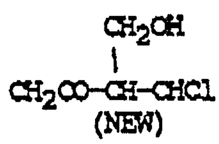<br>(NEW) | 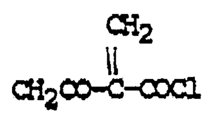 |
| $CNCH_2CN$<br>Malononitrile | 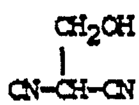<br>(NEW) | 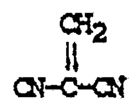<br>(NEW) |
| $CH_3CO$-$CH_2$-$COOC_2H_5$<br>Ethylacetoacetate | 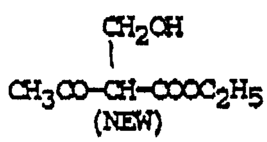<br>(NEW) | 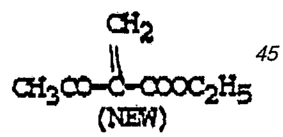<br>(NEW) |

$ClCH_2CO-CH_2-COOC_2H_5$

Ethyl-chloro-aceto-acetate

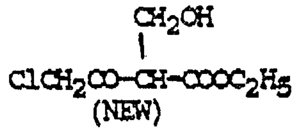

(NEW)

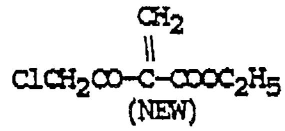

(NEW)

$COCl-CH_2-COOC_2H_5$

Ethyl malonyl chloride

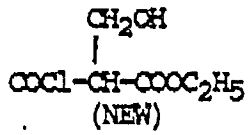

(NEW)

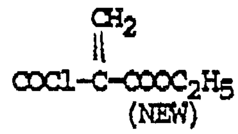

(NEW)

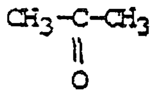

Acetone

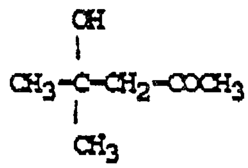

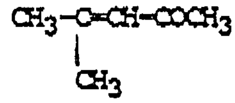

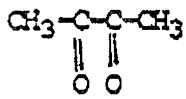

2,3-butanedione

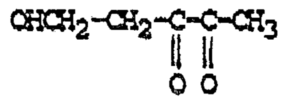

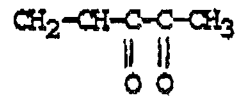

EXAMPLE 1:

This Example describes the preparation of mipk.

A reaction tube made of 316 stainless steel with an inner diameter of 15 mm, a wall thickness of 2 mm and length of 510 mm was charged with 75.0 g of acidic synthetic ion exchange resin sold under the name "Amberlite -IR-118 (H)". Electric heating tape was wrapped directly around the outer surface of the reaction tube to provide heat. The tube was then insulated with 2.5 cm thick fibre glass pipe insulation.

The resin was in the form of bead-like granules having an apparent density of 0.787 g/cm$^3$ and sized 20 to 50 mesh by U.S. Standard Screen.

Ten litres of a mixture of 4-hydroxy-3-methyl-2-butanone (methylol) prepared as described in Example A, phosphoric acid and hydroquinone was initially charged to the supply methylol pre-heating tank. The mixture was 10.0 mL phosphoric acid and 2.5 g hydroquinone per 4.0 L methylol. The supply methylol pre-heating tank, made of 316 stainless steel with 28 cm diameter, 23 cm height and 0.5 cm wall thickness, was heated with an electric hot plate with thermostat control, so that the temperature of the contents was maintained at 80°C. A water-cooled condenser and scrubber filled with Carusorb 200 made by Carus Chemical Company, were used to suppress and absorb fumes of the heated methylol mixture.

The pre-heated methylol mixture then was pumped at a rate of 1.5 L/hr. through a 610 cm long coil of 20 gauge, welded, annealed stainless steel tubing which was immersed in a hot oil bath. The pump used was stainless steel with a Teflon (trademark) diaphragm, Series 500 metering pump made by Neptune Chemical Pump Company. The hot oil bath consisted of 10 L of Therminol 55 (trademark) heat transfer oil made by Monsanto, placed in a porcelain steel vessel. The temperature of the oil was maintained at 150°C by a thermostatically controlled electric hot plate.

The methylol mixture then was passed to the inlet of the reactor tube where temperature and pressure were measured. Operating inlet conditions were 110°C and 100 psi. Exterior reaction tube temperature was maintained at 150°C. Operating outlet temperature and pressure were measured to be 120°C and 85 psi. The thermometers used were bi-metallic 316 stainless steel. The pressure gauges were Bourdon, tube separated from the process fluid by a 316 stainless steel diaphragm.

Pressure of the system was maintained by using a stainless steel needle valve as a throttling device.

The reaction mixture then entered 300 cm of stainless steel coil, similar to the coil used for heating, immersed in a cold water bath where it was cooled to 20°C.

The reaction product obtained after two hours of operation under constant reaction conditions (temperatures and pressures: as stated; throughput: 1.5 L/hr.) was examined for determination of methyl isopropenyl ketone as follows:

Overall molar conversion of 4-hydroxy-3-methyl-2-butanone to methyl isopropenyl ketone and water was 90%, based on gas chromatograph analysis.

EXAMPLE 2:

Using the same reaction conditions as in Example 1 with some apparatus modifications, a purified methyl isopropenyl ketone was produced on a continuous basis.

The cooling coil after the needle valve was removed and a distillation apparatus installed. The distillation apparatus consisted of a 2 L glass, 3-neck round bottom flask, connected to a glass water-cooled condenser feeding another 2 L glass, 3-neck round bottom flask.

The reaction mixture passed from the outlet from the needle valve to the first 3-neck round bottom flask which was immersed in a hot oil bath, heated to 120° C. The heating fluid used was silicon oil, placed in a steel vessel heated by an electric hot plate. A mercury filled glass thermometer was placed in the middle neck of the flask. The third neck was connected to the water-cooled condenser.

The second 2 L round bottom flask collected the distilled methyl isopropenyl ketone. A glass plug was placed in one of the two side necks while a Carusorb 200 filled scrubber was attached to the other neck. The middle neck was connected to the water-cooled condenser.

The methyl isopropenyl ketone produced by continuous distillation was found to be 98.8% pure by weight, resulting in an overall conversion of 95% based on the 4-hydroxy-3-methyl-2-butanone charge.

EXAMPLE 3:

The procedure and operating conditions described in Example 1 were repeated with the following exceptions:

The reaction tube was loaded with 40.0 g of Amberlite IR-118-(H) and 80.0 g of crushed glass particles of various size and shape as support for the catalyst. The average throughput rate was increased from 1.5 L per hour to 2.1 L per hour.

The reaction product obtained after five hours of constant reaction conditions was found to be 82 wt.% methyl isopropenyl ketone by weight with an overall conversion of 75%.

EXAMPLE 4:

The procedure of Example 1 was repeated with the following exception.

The reaction tube was loaded with 81.0 g of Amberlite IR-118-(H) resin and 9.0 of activated alumina. The activated alumina, originally 4 to 8 mesh by U.S. Standard Screen, was crushed using a mortar and pestle and sized using a metal sieve to result in ground activated alumina with particle size similar to the Amberlite resin used.

The reaction product obtained after two hours of operation at constant reaction conditions (as stated in Example 1) was examined for determination of methyl isopropenyl ketone as follows:

Overall molar conversion of 4-hydroxy-3-methyl-2-butanone to methyl isopropenyl ketone and water was 92 wt.% based on water formation.

EXAMPLE 5:

The procedure of Example 1 was repeated except the reaction tube was loaded with 45.0 g of Amberlite IR-118-(H) resin and 45.0 g of activated alumina prepared as stated in Example 4.

The reaction product obtained after two hours of operation at constant reaction conditions was examined for determination of methyl isopropenyl ketone as follows:

Overall molar conversion of 4-hydroxy-3-methyl-2-butanone to methyl isopropenyl ketone and water was 97.7 wt.% based on water formation.

EXAMPLE 6:

The procedure of Example 2 was repeated except for the following:

The portion of the system from the exit of the reaction tube to the inlet of the distillation flask was wrapped in heating tape. A temperature of 130° C was maintained on the outer surface of this portion of the system.

The distillation flask used was a 1.0 L three-neck round-bottom pyrex flask. This flask was immersed in a silicon oil bath maintained at 130° C.

The same condenser and receiver vessel were used as in Example 2.

The throughput rate was 1.1 L per hour.

The reaction tube was loaded with 45.0 g Amberlite IR-118-(H) and 45.0 g of crushed activated alumina prepared as in Example 5.

The reaction product obtained after 1.5 hours of operation at constant reaction conditions was examined for determination of methyl isopropyl ketone as follows:

Methyl isopropenyl ketone produced by continuous distillation was found to be 95.5% by weight. Overall mass conversion of 4-hydroxy-3-methyl-2-butanone to methyl isopropenyl ketone and water was 96.5 wt.% based on water formation.

EXAMPLE 7:

The procedure of Example 6 was repeated except a vacuum of 28 in Hg was applied on the distillation apparatus and the throughput rate was 0.6 L per hour.

The reaction product obtained after three hours of operation at constant reaction conditions was examined

for determination of methyl isopropenyl ketone.

Methyl isopropenyl ketone produced by continuous reaction of 4-hydroxy-3-methyl-2-butanone and continuous vacuum distillation of the reaction product was found to be 91.4 wt.%. Overall mass conversion of 4-hydroxy-3-methyl-2-butanone to methyl isopropenyl ketone and water was 95.2 wt.% based on water formation.

As may be seen from the data presented in these Examples, high yields of high purity unsaturated compounds can be obtained by using the catalysts and apparatus of the invention. The procedures described may be used to form other unsaturated compounds, as described in the following Examples 8 to 10.

EXAMPLE 8:

This Example describes the production of methyl vinyl ketone.

A reaction tube made of 316 stainless steel $\frac{1}{2}$" nominal schedule 10 pipe, 510 mm long was charged with 85.0 g of a mixture of acidic synthetic ion exchange resin and $Al_2O_3$ in a weight ratio of 1:1.

Twelve litres of a mixture of 3-keto butanol, catalytic amount of phosphoric acid and hydroquinone was initially charged to the supply tank. The mixture was 10 mL of 85% orthophosphoric acid and 2.5 g of hydroquinone per 4.0 L of 3-ketobutanol.)

With the supply tank temperature maintained at 35° C, the warmed 3-ketobutanol mixture then was pumped at a rate of 2.4 L/hr. through the stainless steel coil.

The 3-ketobutanol mixture then passed to the inlet of the reaction tube where the temperature and pressure were measured. The operating inlet conditions were 70° C and 100 psig. The exterior of the reaction tube was wrapped with electric heating tape. The temperature of the exterior surface of the reaction tube was maintained at 115° C during the operation of the system, providing operating outlet conditions of 95° C and 40 psig. The pressure of the system was maintained by using a stainless steel needle valve as a throttling device.

The reaction mixture entered to the liquid-vapour separator, where the temperature was maintained at 90° C. 35% of the ingoing 3-ketobutanol was distilled off continuously as methyl vinyl ketone and water. 10% waste was taken off and 50% was recirculated to the supply tank (5% loss).

The water was separated from MVK by saturating the liquid with sodium chloride. 0.4 L/hr. methyl vinyl ketone was generated, of 98% by weight purity.

EXAMPLE 9:

The procedure of Example 8 was repeated using monomethylolmalonic acid-ethyl ester, prepared as described in Example B.

The parameters employed were:

| | |
|---|---|
| Supply tank temperature: | 50° C |
| Preheater: | 90° C |
| Reactor outside temp.: | 130° C |
| Reactor inside temp.: | 110° C |
| Pressure (inlet): | 100 psi |
| Pressure (outlet): | 60 psi |
| Separator temperature: | 120° C |
| Vacuum: | 7 Hg mm |
| Flowrate: | 1 L/hr. |
| Distillation Temp.: | 90° to 97° C |

The yield of methylene malonic acid ethyl ester was 68%. The residue was not recirculated because it was found to be 1,1,3,3-tetracarbonic acid tetraethylester. An analytical sample of the product was prepared by distilling the crude product three times in high vacuum at 0.1 mm Hg. The refractive index was found to Ap 1.4245, which was identical to that for the material prepared by the method described in Org. Syntheses Coll., vo. 4, p.298 (1963z0).

EXAMPLE 10:

The procedure of Example 8 again was repeated using 3-monomethylol-2,4-pentane-dione, prepared as described in Example C.

The parameters employed were:

| | |
|---|---|
| Supply tank temperature | 50° C |
| Preheater | 85° C |
| Reactor outside temperature | 150° C |
| Reactor inside temperature | 130° C |
| Pressure inlet | 100 psi |
| Pressure outlet | 60 psi |
| Separator temperature | 150° C |
| Vacuum | 5 Hg mm |
| Flowrate | 1 L/hr |
| Distillation temperature | 105° C |

The yield of 3-methylene-2,4-pentanedione, a new compound, was 50%. The residue was not recirculated, because it was an unidentified mixture of polymers and side products. An analytical sample of the crude product was prepared by distilling the material three times in high vacuum (0.1 mmHg) and the material analyzed.

| | | | |
|---|---|---|---|
| Theoretical: $C_6H_8O_2$ | C-64.27% | Found | C-64.39% |
| | H-7.19% | | H-7.01% |
| | O-28.54% | | O-28.60% |

The good correlation of determined carbon, hydrogen and oxygen contents of the product demonstrated that the product was the expected one.

## SUMMARY OF THE DISCLOSURE

In summary of this disclosure, the present invention provides a novel process for dehydrating alkanols to the corresponding unsaturated compounds, includes ketones, nitriles and esters, as well as certain novel compounds. Modifications are possible within the scope of this invention.

### Claims

1. A process for the production of an unsaturated compound, which comprises dehydrating a preformed liquid alkanol of the general formula:

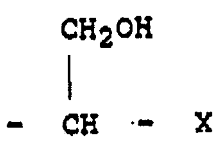

(I)

where X is an activating group for the dehydration reaction in the presence of a solid dehydration catalyst selected from the group consisting of an acid resin, a mixture of acid resin and alumina, alumina, a mixture of alumina and a heavy metal oxide and a mixture of acid resin, alumina and heavy metal oxide.

2. A process as claimed in claim 1, in which X is a carbonyl group or a cyano group.

3. A process as claimed in claim 2, in which the compound of formula (I) has the formula:

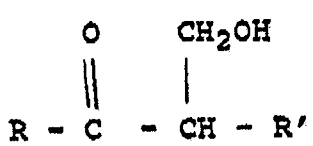

where R represents a lower alkyl group, a halogen atom, an amine group, or an $-OR_3$ group where $R_3$ represents a lower alkyl group, and R′ represents hydrogen, a lower alkyl group, or an $-OR_3$ group, and the product unsaturated compound has the formula:

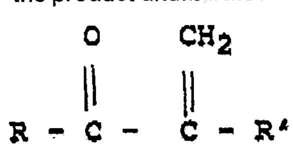

4. A process as claimed in claim 3, in which the product unsaturated compound is methyl isopropenyl ketone or methyl vinyl ketone.

5. A process as claimed in claim 2, in which the compound of formula (I) has the formula:

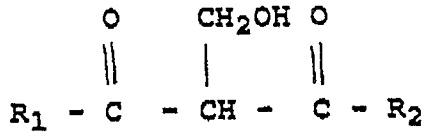

where $R_1$ represents a lower alkyl group, or a halogen atom, an amino group, or an $-OR_3$ group where $R_3$ represents a lower alkyl group, and $R_2$ represents a hydrogen atom, a lower alkyl group, an $-OR_3$ group, a halogen atom or an amino group, and the product unsaturated compound has the formula:

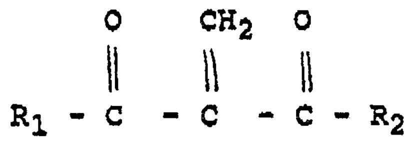

6. A process as claimed in claim 2 in which the compound of formula (I) has the formula:

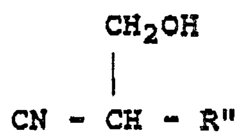

where R″ represents a cyano group, an $R_4CO-$ group in which $R_4$ represents a lower alkyl group, or an $-OR_3$ group in which $R_3$ represents a lower alkyl group and the product unsaturated compound has the formula:

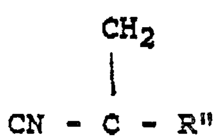

7. A process as claimed in any one of claims 1 to 6, in which the catalyst comprises a mixture of acid resin and alumina in the weight proportions of about 90:10 to about 50:50 resin to alumina.

8. A process as claimed in any one of claims 1 to 6, in which the catalyst comprises a major portion of alumina and a minor portion of a heavy metal oxide and containing about 97.5 to about 99.75 wt.% of alumina.

9. A method as claimed in claim 8, in which the heavy metal oxide is iron oxide, nickel oxide, cobalt oxide, titanium dioxide, vanadium oxides, magnesium oxides, copper oxides, zinc oxide, zirconium oxides, molybdenum oxide, palladium oxide, platinum oxide or tungsten oxide.

10. A method as claimed in any one of claims 1 to 9, in which the dehydration is effected by passing the alkanol in liquid form over a fixed bed of catalyst in an elongate reaction chamber at a temperature in the range of 80° to 200°C.

11. A method as claimed in claim 10, in which the alkanol in liquid form is fed from a source thereof maintained at a temperature of 50° to 90°C and is preheated to a temperature of 110° to 150°C before passing in contact with the fixed catalyst bed.

12. A method as claimed in claim 10 or 11, in which the reaction chamber is maintained under a pressure of 5 to 10 bar.

13. A method as claimed in any one of claims 1 to 12, in which a reaction product mixture from the dehydration is subjected to evaporative separation to remove water and product unsaturated compound from unreacted alkanol, the evaporated unsaturated compound and water are condensed to form two liquid phases, and the two condensed phases are separated to recover product unsaturated compound.

14. A method as claimed in claim 13, in which the evaporative separation is effected at a temperature of 80° to 120°C.

15. A method as claimed in claim 12 or 13, in which the evaporative separation and separation of the condensed phases is effected at atmospheric pressure or below.

16. A method as claimed in any one of claims 1 to 15, which is carried out continuously.

17. A novel compound of the formula:

Y-X-Z

wherein X represents the group

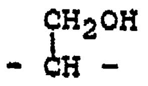 or 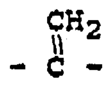

Y represents $R_1CO$-, in which $R_1$ represents a lower alkyl group or a halogen atom, an, or CN- and, (a) when Y is $R_1CO$-, Z represents -CO-$R_2$ in which $R_2$ represents a hydrogen atom, a halogen atom, or an -$OR_3$ group in which $R_3$ represents a lower alkyl group, (b) when Y is -CN, Z represents a cyano group or a $R_4CO$- group in which $R_4$ represents a lower alkyl group or an -$OR_3$ group in which $R_3$ represents a lower alkyl group.

18. A compound as claimed in claim 17, in which Y is $CH_3CO$- and Z is

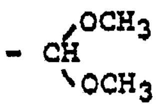 , -COCl, -$COOC_2H_5$ or -$COCH_3$.

-COCl, -$COOC_2H_5$ or -$COCH_3$.

19. A compound as claimed in claim 17, in which Y is CN- and Z is -CN or -$COCH_3$.

20. A compound as claimed in claim 17, in which Z is-$COOC_2H_5$ and Y is $ClCH_2CO$- or ClCO-.

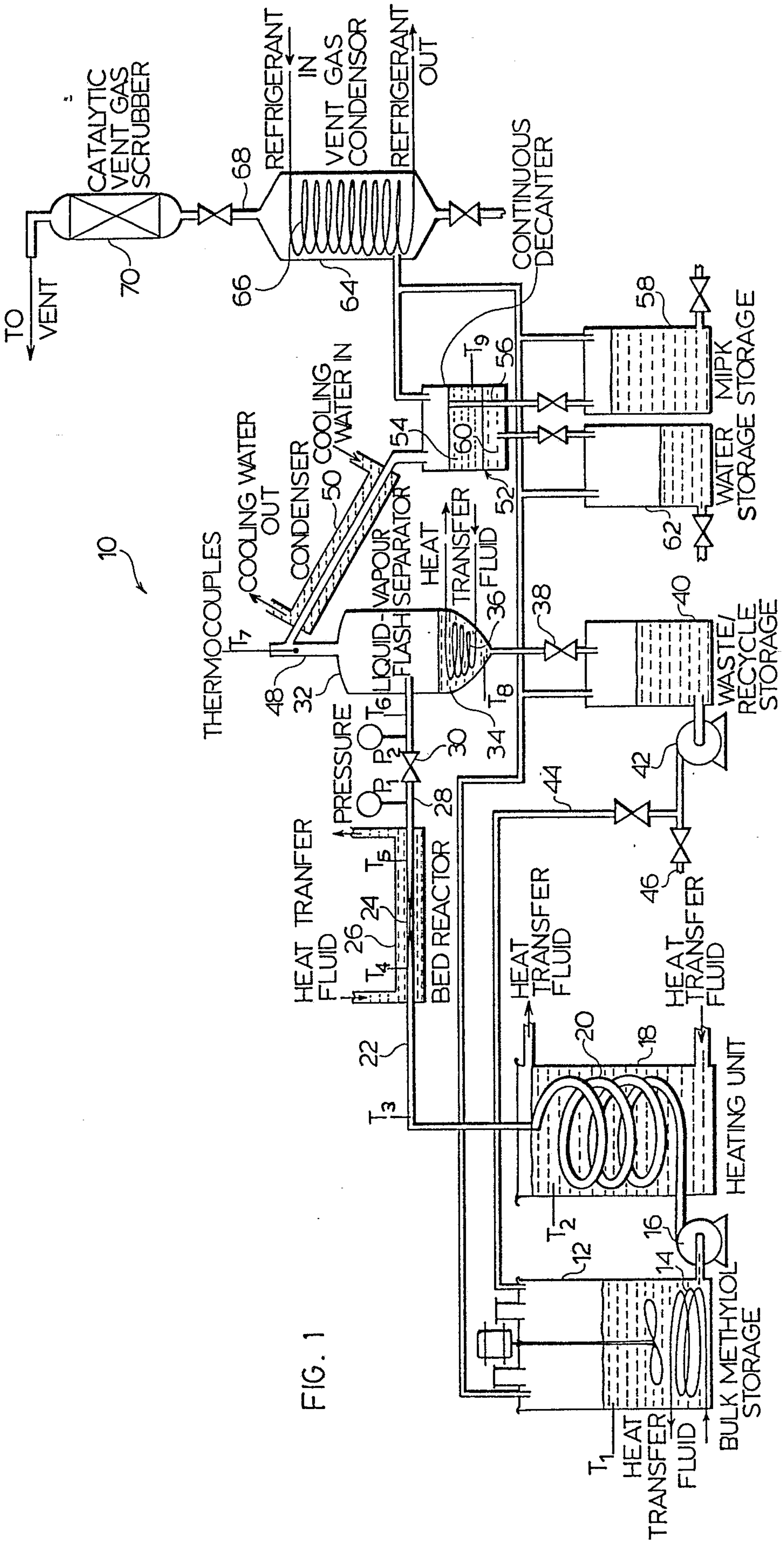
10
TO VENT
70
CATALYTIC VENT GAS SCRUBBER
68
REFRIGERANT IN
VENT GAS CONDENSOR
REFRIGERANT OUT
66
64
CONTINUOUS DECANTER
T9
56
58
MIPK STORAGE
54
60
52
WATER STORAGE
62
COOLING WATER IN
COOLING WATER OUT
CONDENSER
50
THERMOCOUPLES
T7
48
32
LIQUID-VAPOUR FLASH SEPARATOR
HEAT TRANSFER FLUID
36
34
T8
38
40
WASTE/ RECYCLE STORAGE
42
44
46
HEAT TRANSFER FLUID
PRESSURE
P1
P2
T6
30
28
HEAT TRANFER FLUID
26
24
T4
T5
BED REACTOR
22
T3
HEAT TRANSFER FLUID
20
18
HEATING UNIT
T2
12
16
14
BULK METHYLOL STORAGE
T1
HEAT TRANSFER FLUID

FIG. 1